# EUROPEAN PATENT APPLICATION

(11) **EP 2 975 430 A1**
(43) Date of publication of application: **20.01.2016**
(21) Application number: 14184011.6
(22) Date of filing: 09.09.2014
(51) Int. Cl.: G01T 1/164

(54) **APPARATUS AND METHOD FOR CALCULATING CORRECTION FACTOR**

(30) Priority: 15.07.2014 KR 20140089027
(71) Applicant: Korea Institute of Radiological & Medical Sciences, Nowon-gu, Seoul 139-706 (KR)
(72) Inventor: Kim, Jin Su, Nowon-gu, Seoul, 139-780 (KR); Lee, Young Sub, Jung-gu, Daejeon-si, 301-823 (KR); Kim, Hee Joung, Wonju-si, Gangwon-do, 220-710 (KR); Kim, Kyeong Min, Seongbuk-gu, Seoul, 136-856 (KR); Lim, Sang Moo, Gangnam-gu, Seoul, 135-991 (KR)
(74) Representative: V.O.

(57) **Abstract**

Provided is an apparatus and method for calculating a correction factor, the apparatus including: a counter configured to acquire first data indicating a counted value of a first radiation source according to an activity and an energy window width, and to acquire second data indicating a coincidence value of a second radiation source according to an activity and an energy window width; and a calculator configured to calculate a correction factor for correcting a difference of the coincidence value of the second radiation source having occurred in response to a single gamma photon emission of the first radiation source, based on the first data and the second data.

## Description

### CROSS-REFERENCE TO RELATED APPLICATION

This application claims the priority benefit of Korean Patent Application No. 10-2014-0089027, filed on July 15, 2014, in the Korean Intellectual Property Office, the disclosure of which is incorporated herein by reference.

### BACKGROUND

### 1. Field of the Invention

Embodiments of the present invention relate to an apparatus and method for calculating a correction factor to correct gamma rays emitted from a radioisotope for treatment when acquiring a positron emission tomography (PET) image.

### 2. Description of the Related Art

A nuclear medicine test of focus may employ single photon emission computed tomography (SPECT) and positron emission tomography (PET).

Here, the SPECT refers to a method of injecting, into the inside of a body, radioisotopes to which antibodies having properties of binding to a certain type of focus are attached, and detecting, outside the body, and imaging radiation emitted from the radioisotopes guided to the predetermined focus within the body. The SPECT may be advantageous in detecting a focus, for example, a thyroid cancer, involved with a metabolic process of the body, infection, and an abnormal change in the epiphyseal. Also, the SPECT may be effectively employed to suit its purpose since it is possible to acquire a plane image and a tomography image.

The PET refers to a nuclear medicine testing method of injecting, into the inside of a body, radioactive pharmaceutical emitting a positron, and detecting, outside the body, a positron emitted from the radioactive pharmaceutical bound to a focus. The PET may be advantageous in verifying functional information of lesion, for example, the spread or a metabolic process of lesion. Also, the PET may reconfigure a positron detected outside the body into a three-dimensional (3D) image using an exclusive imaging device, for example, a PET scanner. Accordingly, the PET may have a relatively excellent resolution and thus, relatively precisely verify a location, a shape, and a metabolic process of the lesion.

In the recent times, such SPECT and PET have been generally employed to diagnose various types of cancers and also have been known as efficient tests to perform a differential diagnosis on a cancer, clinical staging, an evaluation of recurrence, and a determination as to the treatment effectiveness. In addition, a receptor image or a metabolic image used for evaluating heart disease, brain disease, and brain functions may be acquired using the SPECT and the PET.

### SUMMARY

According to an aspect of the present invention, there is provided a method of calculating a correction factor, the method including: acquiring first data indicating a counted value of a first radiation source according to an activity and an energy window width; acquiring second data indicating a coincidence value of a second radiation source according to an activity and an energy window width; and calculating a correction factor for correcting a difference of the coincidence value of the second radiation source having occurred in response to a single gamma photon emission of the first radiation source, based on the first data and the second data.

According to another aspect of the present invention, there is provided an apparatus for calculating a correction factor, the apparatus including: a counter configured to acquire first data indicating a counted value of a first radiation source according to an activity and an energy window width, and to acquire second data indicating a coincidence value of a second radiation source according to an activity and an energy window width; and a calculator configured to calculate a correction factor for correcting a difference of the coincidence value of the second radiation source having occurred in response to a single gamma photon emission of the first radiation source, based on the first data and the second data.

### BRIEF DESCRIPTION OF THE DRAWINGS

These and/or other aspects, features, and advantages of the invention will become apparent and more readily appreciated from the following description of embodiments, taken in conjunction with the accompanying drawings of which:
Figure 1 illustrates a characteristic of radiation.
Figure 2 illustrates a theory of a single photon emission computed tomography (SPECT) device.
Figure 3 illustrates a theory of a positron emission tomography (PET) device.
Figure 4 illustrates a decay scheme of 1-131 radioisotope.
Figure 5 is a table showing a characteristic of radioisotope for nuclear medicine.
Figure 6 illustrates a configuration of a correction factor calculating apparatus according to an embodiment.
Figure 7 is a table showing first data and second data according to an embodiment.
Figure 8 is a table showing an adding coincidence value based on an activity and an energy window width according to an embodiment.
Figure 9 is a graph showing total coincidence values with respect to an activity calculated by a calculator of a correction factor calculating apparatus according to an embodiment.
Figures 10 through 15 are graphs individually showing the graph of figure 9 based on an energy window width.
Figures 16 through 21 are graphs individually showing an adding coincidence value ratio (%) value with respect to an activity based on an energy window width.
Figure 22 is a flowchart illustrating a method of calculating a correction factor according to an embodiment.

### DETAILED DESCRIPTION

Hereinafter, embodiments of the present invention will be described with reference to the accompanying drawings.

When it is determined detailed description related to a related known function or configuration they may make the purpose of the present invention unnecessarily ambiguous in describing the present invention, the detailed description will be omitted here. Also, terms used herein are defined to appropriately describe the exemplary embodiments of the present invention and thus may be changed depending on a user, the intent of an operator, or a custom. Accordingly, the terms must be defined based on the following overall description of this specification. Like reference numerals refer to like elements throughout.

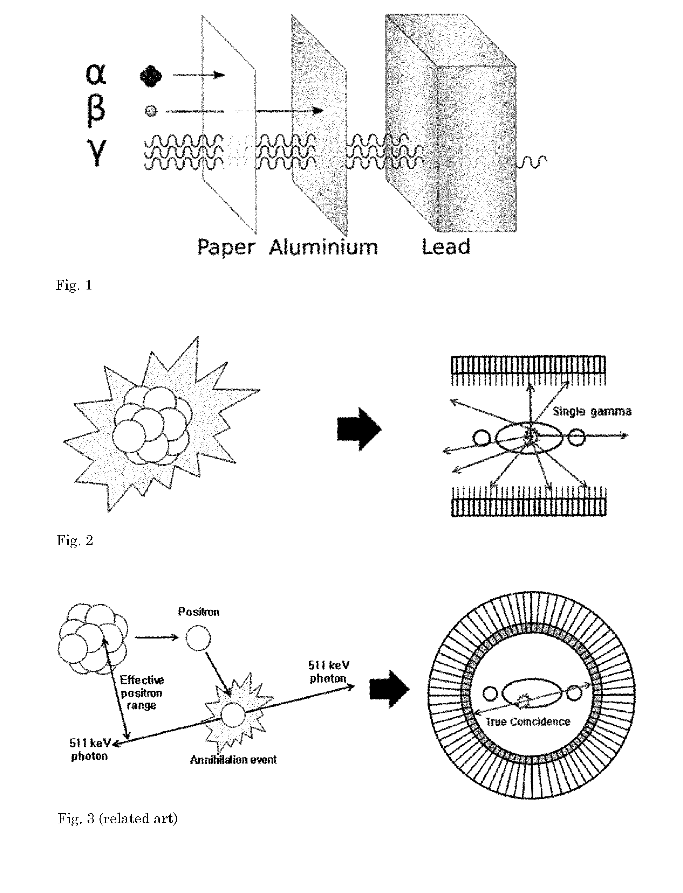

Figure 1 illustrates a characteristic of radiation.

Referring to figure 1, among radiation rays, α ray is incapable of penetrating even paper, β ray is capable of penetrating paper, however, incapable of penetrating a thin aluminum film, and γ ray is capable of penetrating the thin aluminum film and also capable of partially penetrating even thick lead. Due to such a difference among characteristics of radiation rays, in a medical diagnostic device such as a positron emission tomography (PET) device and a single photon emission computed tomography (SPECT) device, β ray emitted from a radiation source injected into a target subject, for example, a human body and an animal, may not penetrate the target subject that is, may not come out of the target subject. On the other hand, γ ray may penetrate the target subject, that is, may come out of the target subject. Accordingly, β ray may be used for treatment and γ ray may be used for a test to verify the treatment effectiveness.

Figure 2 illustrates a theory of a SPECT device.

Referring to figure 2, the SPECT device may detect a single gamma photon emitted from a radiation source. Depending on embodiments, the SPECT device may use 1-131 radioisotope as the radiation source. Here, the 1-131 radioisotope may emit both β ray and γ ray.

Figure 3 illustrates a theory of a PET device.

Referring to figure 3, the PET device may perform coincidence on two electric waves, for example, photons emitted from a radiation source. Depending on embodiments, the PET device may use F-18 radioisotope as the radiation source.

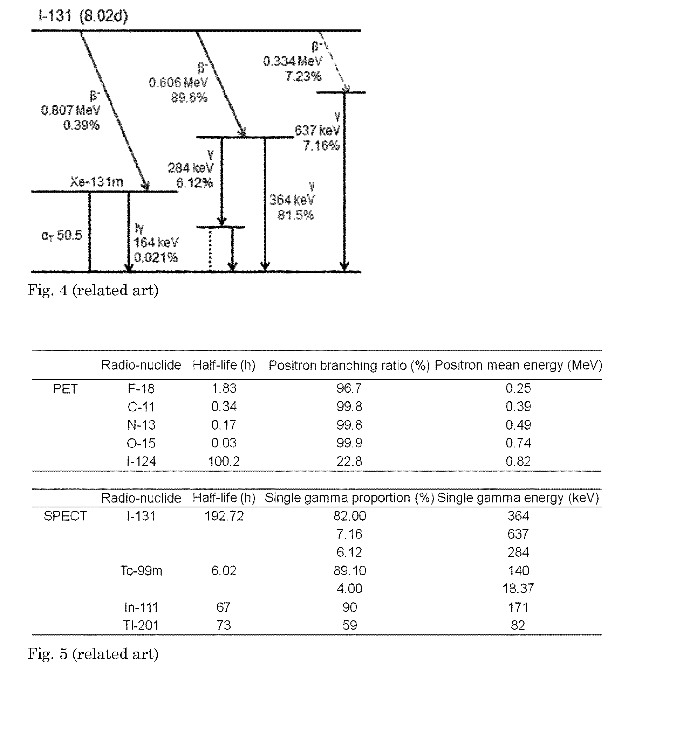

Figure 4 illustrates a decay scheme of 1-131 radioisotope, and figure 5 is a table showing a characteristic of radioisotope for nuclear medicine.

Referring to figures 4 and 5, the 1-131 radioisotope has a relatively long half-life compared to the F-18 radioisotope. Meanwhile, it can be verified from the following table that the PET device has a relatively excellent sensitivity and resolution compared to the SPECT device.

| Modality | Sensitivity | Resolution | Contrast |
|---|---|---|---|
| PET | High | 3 ∼ 4 mm | Medium |
| SPECT | Medium | 8 ∼ 12 mm | Low |

Accordingly, in a case in which a test can be performed with the PET device having a relatively excellent sensitivity and resolution while performing treatment using β ray by injecting 1-131 radioisotope into a target subject, the test may be performed faster compared to a case of using only the SPECT device. However, referring to figures 4 and 5, the half-life of the 1-131 radioisotope is about 192.72 hours, for example, about eight days and thus, very long. Accordingly, inspecting the treatment effectiveness by injecting F-18 radioisotope immediately after treatment may not achieve an explicit result due to interference of 1-131 radioisotope.

Accordingly, when inspecting the treatment effectiveness through the PET device using a radiation source such as F-18 radioisotope, there is a need for a precise diagnosis to correct a PET image by measuring a level of effect of a single gamma photon emission from another radiation source such as I-131 radioisotope against the coincidence of F-18 and by applying the measured level of effect. Accordingly, embodiments of the present invention propose a method of calculating a correction factor used to correct a PET image.

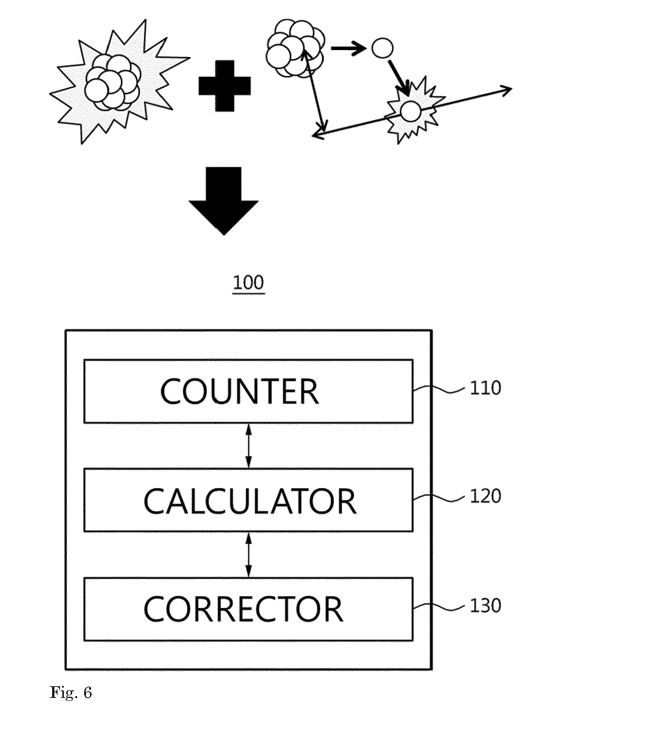

Figure 6 illustrates a configuration of a correction factor calculating apparatus 100 according to an embodiment.

Referring to figure 6, the correction factor calculating apparatus 100 may include a counter 110 and a calculator 120. The correction factor calculating apparatus 100 may be embedded into a PET device as one of modules and thereby operate.

The counter 110 may acquire first data indicating a counted value of a first radiation source according to an activity and an energy window width. Depending on embodiments, the first radiation source may be a radiation source used by a SPECT device. For example, the first radiation source may be 1-131 radioisotope.

The counter 110 may acquire second data indicating a coincidence value of a second radiation source according to an activity and an energy window width. Depending on embodiments, the second radiation source may be a radiation source used by a PET device. For example, the second radiation source may be F-18 radioisotope.

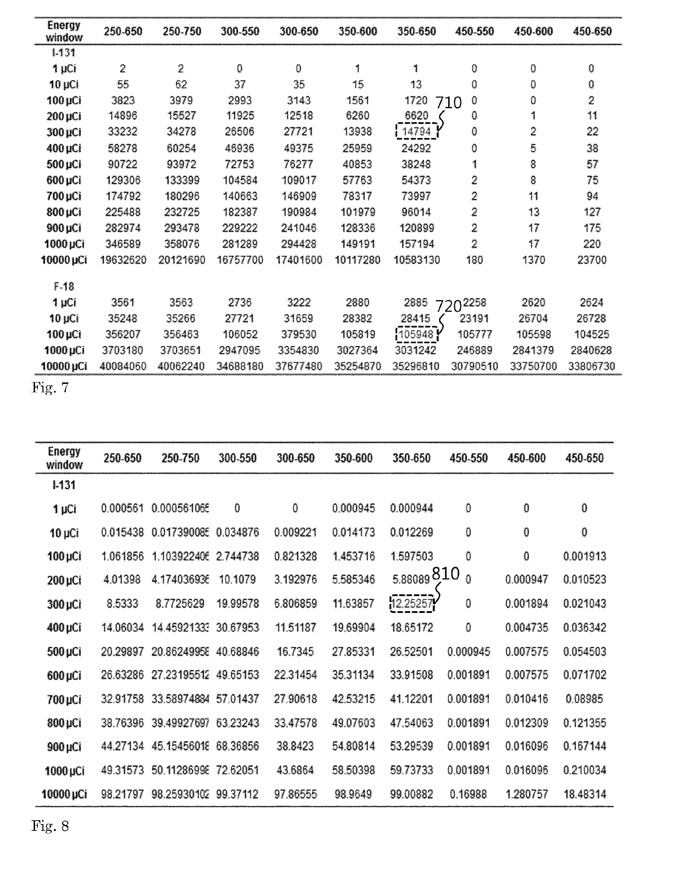

Figure 7 is a table showing first data and second data according to an embodiment.

Referring to figure 7, the counter 110 of the correction factor calculating apparatus 100 may acquire a counted value of a radiation source according to a change in an activity and a change in an energy window width.

The counter 110 may acquire a counted value of 1-131 radioisotope. For example, the counter 110 may acquire "14794" indicated in a dotted box 710 as a counted value of 1-131 radioisotope according to an activity of 300 µCi and an energy window width of 350 to 650 keV.

The counter 110 may acquire a coincidence value of F-18 radioisotope. For example, the counter 110 may acquire "105948" indicated in a dotted box 720 as a coincidence value of F-18 radioisotope according to an activity of 100 µCi and an energy window width of 350 to 650 keV.

Depending on embodiments, the counter 110 may acquire a counted value by driving a simulation program. For example, the counter 110 may acquire a simulation result value based on the following conditions:
- Simulation environment: Siemens INVEON PET scanner, GATE ver. 6.2
- Line source: positioning a dotted line source (1mm) on the center of the scanner (in the air)
- Activity: 1, 10, 100, 1000, 10000 µCi (each 100 µCi for 100 to 1000 µCi)
- Time: 1 sec
- Analysis method: verifying a coincidence value by acquiring ASCII data

The calculator 120 may calculate a correction factor based on the first data and the second data. Here, the correction factor may be used to correct a difference of the coincidence value of the second radiation source having occurred in response to a single gamma photon emission of the first radiation source, based on the first data and the second data. For example, in a case in which both 1-131 radioisotope and F-18 radioisotope are injected into a target subject, when the PET device performs coincidence on gamma rays emitted from the F-18 radioisotope, noise by a single gamma photon emitted from the 1-131 radioisotope may be added. In this case, the calculator 120 of the correction factor calculating apparatus 100 may calculate the correction factor to acquire a PET image in which the added noise is removed.

Depending on embodiments, when the second radiation source has a predetermined activity, for example, when the F-18 radioisotope has an activity of 100 µCi, the calculator 120 may calculate an adding or subtracting coincidence value of the second radiation source according to a change in an activity of the first radiation source. Referring to figure 8, when the F-18 radioisotope has an activity of 100 µCi, the calculator 120 may calculate an adding or subtracting coincidence value according to a change in the activity of the 1-131 radioisotope, based on an energy window width. For example, when the F-18 radioisotope has an activity of 100 µCi, the calculator 120 may calculate 12.25257 indicated in a dotted box 810 as an adding coincidence ratio (%) by the 1-131 radioisotope at an activity of 300 µCi and an energy window width of 350 to 650 keV.

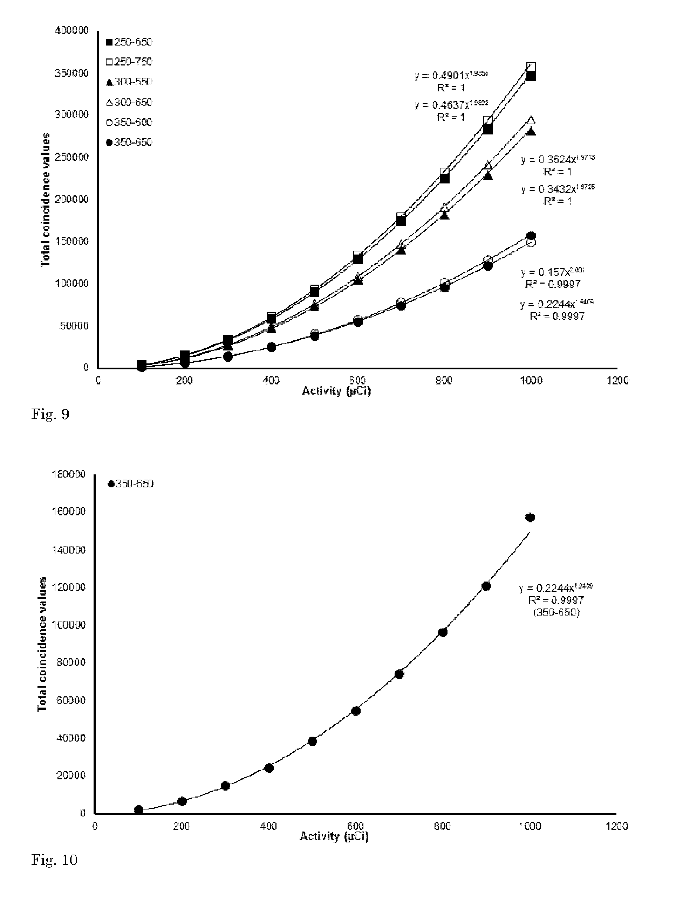

Figure 9 is a graph showing total coincidence values with respect to an activity calculated by the calculator 120 of the correction factor calculating apparatus 100 according to an embodiment.

Referring to figure 9, the calculator 120 may calculate an equation expressing a relationship between an activity and total coincidence values and a correlation coefficient R.

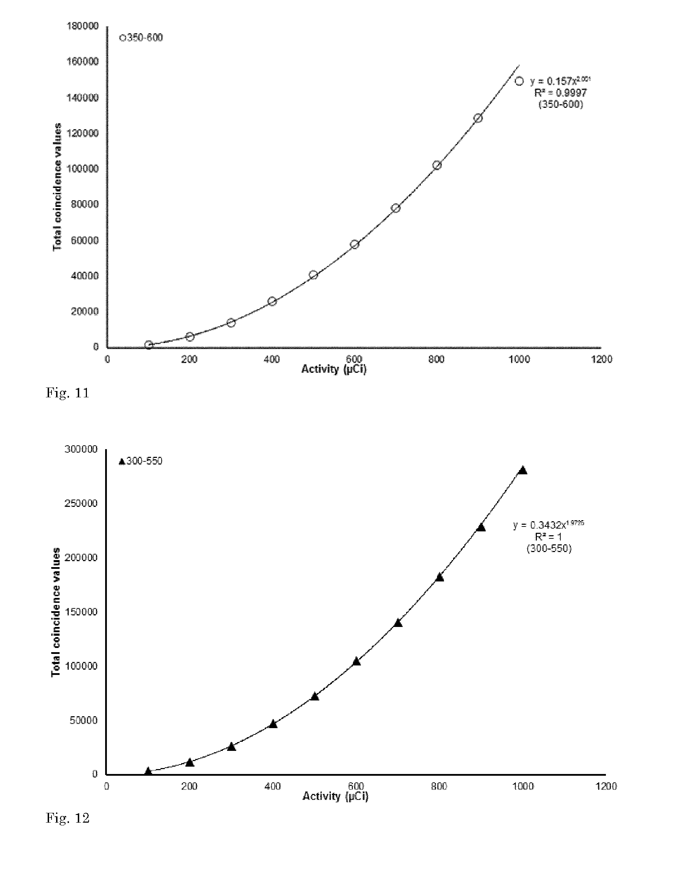

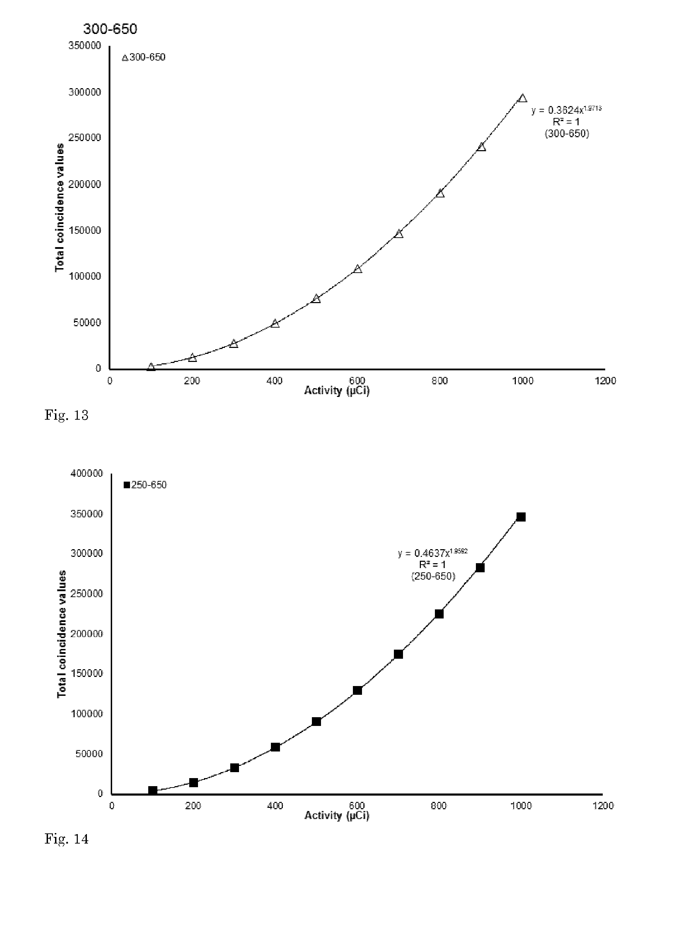

Also, figures 10 through 15 are graphs individually showing the graph of figure 9 based on an energy window width.

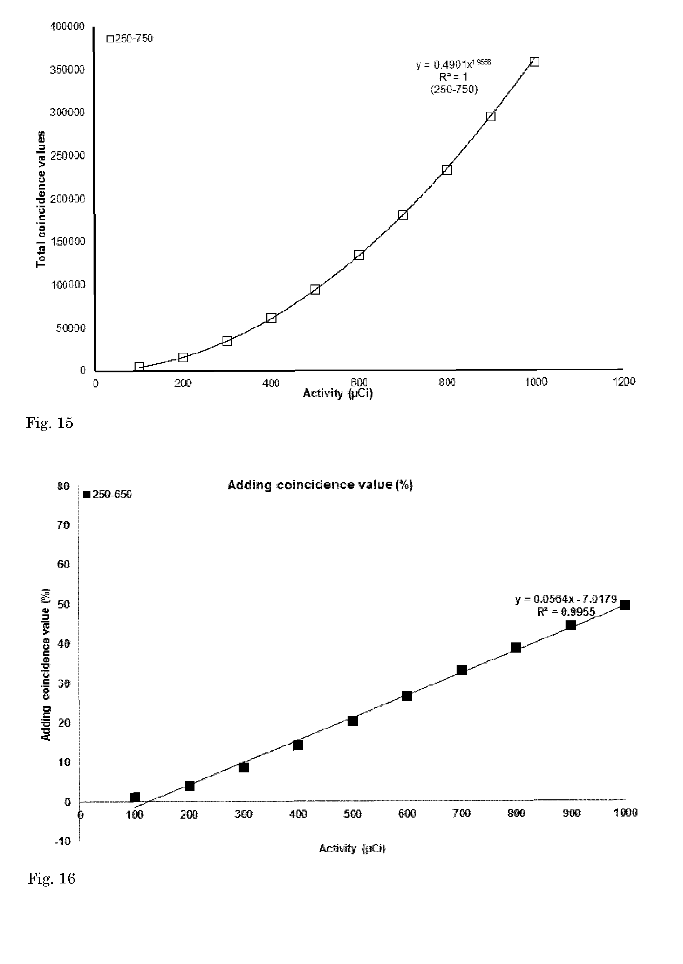

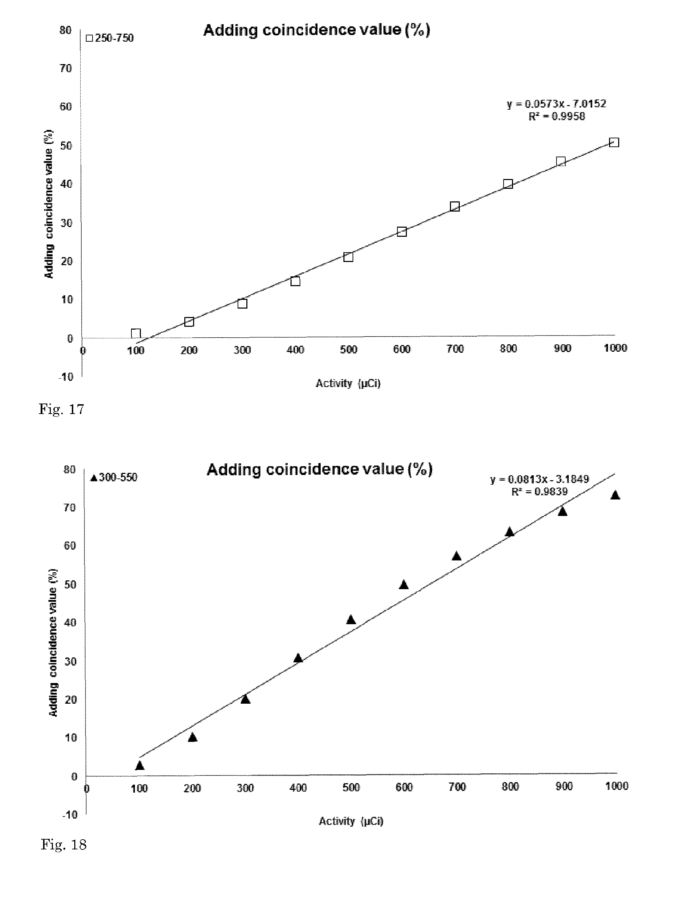

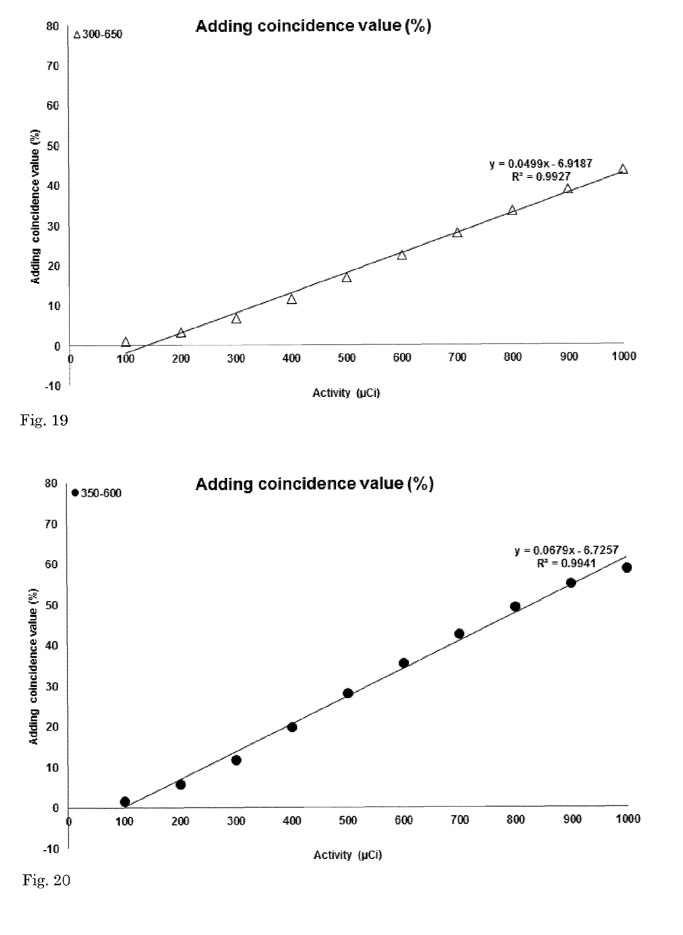

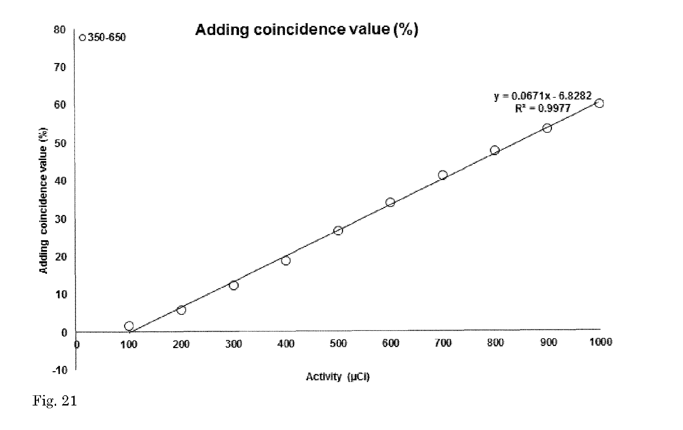

Also, figures 16 through 21 are graphs individually showing an adding coincidence value ratio (%) value with respect to an activity, based on an energy window width.

Referring again to figure 6, the correction factor calculating apparatus 100 may further include a corrector 130.

The corrector 130 may correct a PET image using the calculated correction factor. Accordingly, the PET device may acquire an image in which noise having occurred due to a single gamma photon is removed through the correction factor calculating apparatus 100.

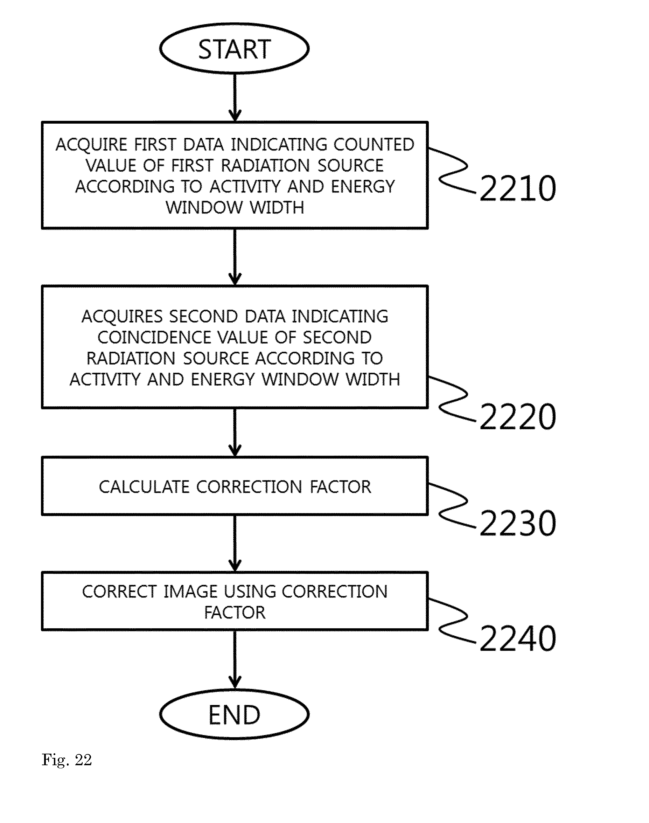

Figure 22 is a flowchart illustrating a method of calculating a correction factor according to an embodiment.

Referring to figure 22, in operation 2210, the correction factor calculating method acquires first data indicating a counted value of a first radiation source according to an activity and an energy window width. Depending on embodiments, the first radiation source may be a radiation source used by a SPECT device. For example, the first radiation source may be 1-131 radioisotope.

In operation 2220, the correction factor calculating method acquires second data indicating a coincidence value of a second radiation source according to an activity and an energy window width. Depending on embodiments, the second radiation source may be a radiation source used by a PET device. For example, the second radiation source may be F-18 radioisotope.

In operation 2230, the correction factor calculating method calculates a correction factor based on the first data and the second data. Here, the correction factor may be used to correct a difference of the coincidence value of the second radiation source having occurred in response to a single gamma photon emission of the first radiation source, based on the first data and the second data. For example, in a case in which both 1-131 radioisotope and F-18 radioisotope are injected into a target subject, when the PET device performs coincidence on gamma rays emitted from the F-18 radioisotope, noise by a single gamma photon emitted from the 1-131 radioisotope may be added. In this case, the correction factor calculating method may calculate the correction factor to acquire a PET image in which the added noise is removed.

Depending on embodiments, when the second radiation source has a predetermined activity, for example, when the F-18 radioisotope has an activity of 100 µCi, the correction factor calculating method may calculate an adding or subtracting coincidence value of the second radiation source according to a change in an activity of the first radiation source.

Depending on embodiments, the correction factor calculating method may further include operation 2240 of correcting a PET image using the corrected correction factor. Accordingly, the PET device may acquire an image in which noise having occurred due to a single gamma photon is removed through a correction factor calculating apparatus

The above-described embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as floptical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described embodiments of the present invention, or vice versa.

Although a few embodiments of the present invention have been shown and described, the present invention is not limited to the described embodiments. Instead, it would be appreciated by those skilled in the art that changes may be made to these embodiments without departing from the principles and spirit of the invention, the scope of which is defined by the claims and their equivalents.

## Claims

1. A method of calculating a correction factor, the method comprising:
acquiring first data indicating a counted value of a first radiation source according to an activity and an energy window width;
acquiring second data indicating a coincidence value of a second radiation source according to an activity and an energy window width; and
calculating a correction factor for correcting a difference of the coincidence value of the second radiation source having occurred in response to a single gamma photon emission of the first radiation source, based on the first data and the second data.

2. The method of claim 1, further comprising:
correcting a positron emission tomography (PET) image using the calculated correction factor.

3. The method of claim 1, wherein the first radiation source is a radiation source used by a single photon emission computed tomography (SPECT) device.

4. The method of claim 1, wherein the first radiation source is an I-131 radioisotope.

5. The method of claim 1, wherein the second radiation source is a radiation source used by a PET device.

6. The method of claim 1, wherein the second radiation source is an F-18 radioisotope.

7. A non-transitory computer-readable medium storing a program to implement the method of claim 1.

8. An apparatus for calculating a correction factor, the apparatus comprising:
a counter configured to acquire first data indicating a counted value of a first radiation source according to an activity and an energy window width, and to acquire second data indicating a coincidence value of a second radiation source according to an activity and an energy window width; and
a calculator configured to calculate a correction factor for correcting a difference of the coincidence value of the second radiation source having occurred in response to a single gamma photon emission of the first radiation source, based on the first data and the second data.

9. The apparatus of claim 8, further comprising:
a corrector configured to correct a positron emission tomography (PET) image using the corrected correction factor.
